(19) Europäisches Patentamt
European Patent Office
Office européen des brevets

(11) Veröffentlichungsnummer: **0 252 389**
**B1**

# (12) EUROPÄISCHE PATENTSCHRIFT

(45) Veröffentlichungstag der Patentschrift: **11.10.89**

(51) Int. Cl.⁴: **C07C 69/16**, C07C 69/28

(21) Anmeldenummer: **87109255.7**

(22) Anmeldetag: **26.06.87**

(54) **1,1-Dialkoxy-2-methyl-4,4- diacyloxy-2-butene.**

(30) Priorität: **05.07.86 DE 3622601**

(43) Veröffentlichungstag der Anmeldung:
**13.01.88 Patentblatt 88/2**

(45) Bekanntmachung des Hinweises auf die Patenterteilung:
**11.10.89 Patentblatt 89/41**

(84) Benannte Vertragsstaaten:
**CH DE FR GB LI NL**

(56) Entgegenhaltungen:
**EP-A- 0 005 452**
**EP-A- 0 162 384**

(73) Patentinhaber: **BASF Aktiengesellschaft, Carl-Bosch-Strasse 38, D-6700 Ludwigshafen(DE)**

(72) Erfinder: **Schulz, Bernhard, Dr., Kurpfalzring 28, D-6830 Schwetzingen(DE)**
Erfinder: **Fischer, Rolf, Dr., Bergstrasse 98, D-6900 Heidelberg(DE)**

**EP 0 252 389 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 1,1-Dialkoxy-2-methyl-4,4-diacyloxy-2-butene der allgemeinen Formel I

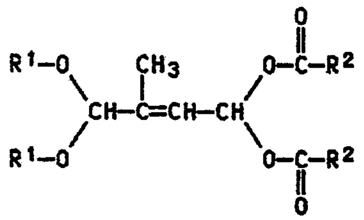

(I),

in der
$R^1$ einen Alkylrest mit 1 bis 5 C-Atomen, vorzugsweise 1 bis 3 C-Atomen bedeutet oder aber beide $R^1$ zusammen einen Ethylen- oder Propylenrest, der noch durch $C_1$- bis $C_4$-Alkylreste substituiert sein kann, vorzugsweise einen Ethylenrest oder einen 2,2-Dimethyl-propylenrest bedeuten und
$R^2$ für einen Alkylrest mit 1 bis 5 C-Atomen, vorzugsweise 1 bis 3 C-Atomen oder einen Cycloalkylrest mit 5 bis 7 C-Atomen, oder einen aromatischen Rest, vorzugsweise eine Phenylgruppe steht.

Die neuen Verbindungen der Formel I sind wertvolle Bausteine für die Synthese von Terpenen und Carotinoiden.

Man kann sie auf recht einfache Weise aus den nach dem Verfahren der EP 68 372 neuerdings recht gut zugänglichen 2-Methyl-4,4-diacyloxy-2-butenalen der allgemeinen Formel II

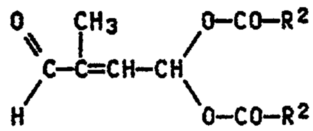

(II)

herstellen.

Beispielsweise kann die Herstellung von I durch Umsetzen von II mit Alkanolen bzw. Alkandiolen oder Orthoameisensäureestern in an sich bekannter Weise erfolgen, so daß sich detaillierte Ausführungen erübrigen. Aus Houben-Weyl, "Methoden der organischen Chemie", 4. Auflage, Band 6/3, Seite 222, ist bekannt, daß bei der Acetalisierung von Aldehyden und Ketonen mit Orthoestern Katalysatoren anwesend sein müssen. Auf Seite 224 wird darauf hingewiesen, daß die Umsetzung von $\alpha,\beta$-ungesättigten Aldehyden mit Orthoestern entweder ganz ohne Alkoholzusatz oder höchstens in Gegenwart von kleinen Mengen Alkohole durchgeführt werden muß. Andernfalls würde eine Anlagerung von Alkohol an die Doppelbindung erfolgen.

Wie das Beispiel der Acetalisierung von Crotonaldehyd zeigt (S. 224), sind zur Erzielung guter Ausbeuten einige Tage Reaktionszeit in Anwesenheit eines Katalysators und Abwesenheit von Alkohol notwendig. Es war daher nicht zu erwarten, daß die Acetalisierung der 2-Methyl-4,4-diacyloxy-2-butenale II glatt und in guten Ausbeuten erfolgen wurde, ohne daß die Doppelbindungen oder die als relativ labil bekannten Acylalfunktionen durch den als Lösungsmittel verwendeten Alkohol angegriffen würden. Dies gilt ganz besonders, da das Molekül II eine Doppelbindung enthält, die in Konjugation zu den Acylalresten steht.

Von den neuen Verbindungen der Formel I sind die Dimethyl-, Diethyl-, Dipropyl-, Ethylen-, Propylen- und 2,2-Dimethyl-propylenacetale des 2-Methyl-4,4-diacetoxy-, 2-Methyl-4,4-dipropionyloxy-, 2-Methyl-4,4-dicyclohexyloxy- und 2-Methyl-4,4-dibenzoyloxy-2-butenals von besonderem Interesse. Sie lassen sich sehr vorteilhaft bei der Knüpfung von Kohlenstoff-Kohlenstoff-Doppelbindungen durch Umsetzen von Carbonylverbindungen mit Phosphoryliden (Wittig-Reaktion) als $C_5$-Bausteine verwenden. Ihre Bedeutung für die Herstellung von Terpenen und Carotinoiden liegt im wesentlichen darin, daß die Acylalgruppe in I bei der Durchführung der Wittig-Reaktion unter den Reaktionsbedingungen zu den Aldehydgruppen verseift werden, während die Acetalgruppen unter den basischen Bedingunge der Wittig-Reaktion nicht angegriffen werden. Durch die hierbei in situ entstehenden 3-Methyl-4,4-dialkoxy-2-butenale können bei den Umsetzungen mit Phosphoryliden in das neuentstehende Olefin selektiv die Reste

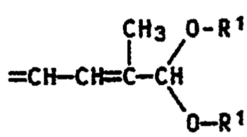

eingeführt werden.

So gelingt es beispielsweise, das aus Vitamin-A zugängliche Triphenylphosphonium-Salz mit 1,1-Dimethoxy-2-methyl-4,4-diacetoxy-2-buten in Gegenwart von Methanol, Wasser und Magnesiumhydroxid mit sehr guten Ausbeuten zu dem entsprechenden $C_{25}$-Dimethylacetal umzusetzen.

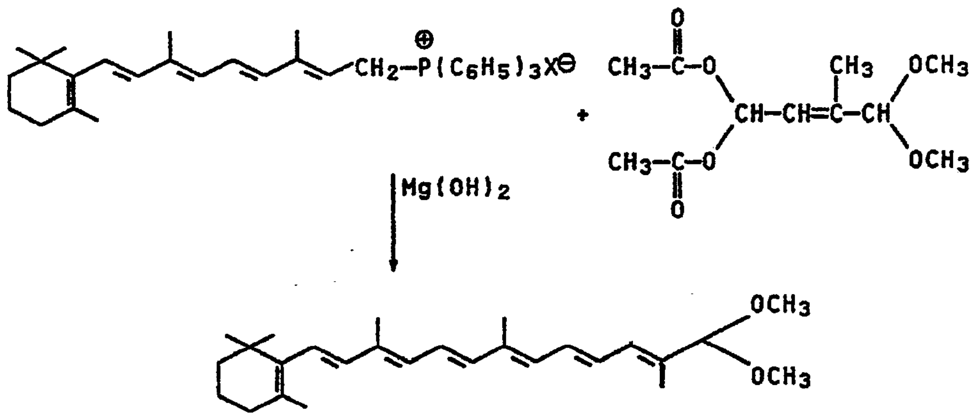


Die neuen 1,1-Dialkoxy-2-methyl-4,4-diacyloxy-2-butene I besitzen bei ihrer Verwendung als Terpenbausteine den Vorteil, daß für die Abspaltung der Acylal-Schutzgruppe keine gesonderte Hydrolyse-Stufe notwendig ist. Im Gegensatz zu den nur unter sauren Bedingungen entfernbaren Acetalschutzgruppen erfolgt die Verseifung der Acylalgruppen unter den Bedingungen der Wittig-Reaktion.

<u>Beispiel 1</u>

a) 1,1-Dimethoxy-2-methyl-4,4-diacetoxy-2-buten

Eine Lösung von 25 g 2-Methyl-4,4-diacetoxy-2-butenal und 20 g o-Ameisensäuretrimethylester in 95 ml Methanol wurde 23 Stunden (h) lang bei 40°C gerührt. Nach Abziehen von Methanol, Ameisensäuremethylester und nicht umgesetztem o-Ameisensäuretrimethylester am Rotationsverdampfer wurden durch fraktionierende Destillation 23,6 g 1,1-Dimethoxy-2-methyl-4,4-diacetoxy-2-buten (84% E, 16% Z) vom Siedepunkt 92 bis 94°C/0.3 mbar erhalten (Ausbeute 77%, bez. eingesetztes 2-Methyl-4,4-diacetoxy-2-butenal).

$^1$H–NMR-Spektren (Lösungsmittel $CDCl_3$, TMS als innerer Standard):

<u>E-1,1-Dimethoxy-2-methyl-4,4-diacetoxy-2-buten</u>:
$\delta$ = 1,80 (d, I = 1 Hz, 3H), 2,09 (S, 6H), 3,29 (S, 6H), 4,53 (S,1H), 5,70 (d breit, I = 8 HZ, 1H), 7,40 (d, I = 8 Hz, 1H)

<u>Z-1,1-Dimethoxy-2-methyl-4,4-diacetoxy-2-buten</u>:
$\delta$ = 1,75 (d, I = 1 Hz, 3H), 2,09 (S, 6H), 3,35 (S, 6H), 5,08 (S, 1H), 5,49 (d breit, I = 8 HZ, 1H), 7,55 (d, I = 8 Hz, 1H), 7,55 (d, I = 8 Hz, 1H)

b) 1,1-[2',2'-Dimethyl-propylen-1',3'-dioxy]-2-methyl-4,4-diacetoxy-2-buten

Durch Umsetzen von 2-Methyl-4,4-diacetoxy-2-butenal mit Neopentylglykol in Gegenwart von p-Toluolsulfonsäure wurde 1,1-[2',2'-Dimethyl-propylen-1',3'-dioxy]-2-methyl-4,4-diacetoxy-2-buten mit folgenden physikalischen Daten erhalten:

Siedepunkt: 126-130°C/0,8 mbar, $n^2\theta$ = 1,4608

$^1$H-NMR-Spektrum (Lösungsmittel $CDCl_3$, TMS als innerer Standard): $\delta$ = 0,74 (s, 3H), 1,20 (s, 3H), 1,86 (d, 3H), 2,08 (s, 6H), 3,55 (m, 4H), 4,68 (s, 1H), 5,72 (d, J = ca. 8Hz, 1H), 7,41 (d, J = ca. 8 Hz, 1H).

<u>Anwendungsbeispiele</u>

a) Herstellung von β-Apo-12'-carotinal

In einem 1 l-Dreihalskolben wurden 23,4 g (0,4 mol) Magnesiumhydroxid und 200 ml einer Mischung aus 6 Teilen Methanol und 4 Teilen Wasser vorgelegt. Unter Rühren werden bei Raumtemperatur (RT) 31,8 g 93 %iges 1,1-Dimethoxi-2-methyl-4,4-diacetoxi-2-buten zugegeben. Dann wurden innerhalb von 30 Minuten (Min.) 200 ml einer Lösung von Axerophtyl-triphenylphosphoniumhydrogensulfat in Methanol zugetropft, die aus 32,8 g Vitamin-A-acetat hergestellt worden war. Anschließend wurde das Reaktionsgemisch noch 5 h bei 50°C gerührt. Dann wurden 500 ml Heptan, 400 ml 20 %ige wäßrige Schwefelsäure und 400 ml Methanol zugegeben und 30 Min. bei 50°C gerührt. Die Unterphase wurde abgetrennt und die Oberphase dreimal mit 250 ml 60 %igem Methanol gewaschen. Die Oberphase wurde eingeengt. Man erhielt 31 g eines gelb-roten Öles ($E_1^1$ = 1680, $\lambda$ max. 410 nm). Die Ausbeute betrug ca. 89 % an rohem β-Apo-12'-carotinal. Das Rohprodukt wurde ohne weitere Reinigung zum β-Apo-8'-carotinal umgesetzt.

b) Herstellung von β-Apo-8'-carotinal

31 g eines gemäß a) erhaltenen rohen β-Apo-12'-carotinals wurden in 80 ml Heptan aufgenommen. Zu dieser Lösung wurden unter Rühren bei RT gleichzeitig 35 ml einer 30 %igen Natriummethylat-Lösung in Methanol und 110 ml einer Lösung von 47 g (0,11 mol) 4,4-Dimethoxi-3-methyl-2-butenyl-triphenylphosphoniumchlorid in Methanol zugegeben. Die Temperatur stieg dabei auf 45°C an. Anschließend wurde 1 h unter Rückfluß zum Sieden erhitzt. Zur Aufarbeitung wurde mit 120 ml Heptan versetzt und bei 50°C mit 100 ml Wasser und dann zweimal mit 250 ml 60 %igem Methanol gewaschen. Nach dem Eindampfen erhielt man 40 g rohes β-Apo-8'-carotinal-dimethylacetal. Dieses Acetal wurde in 50 ml Heptan und 250 ml Isopropanol aufgenommen und mit 20 ml einer 32 %igen Schwefelsäure versetzt und 12 h bei RT gerührt. Das entstandene Kristallisat wurde abgesaugt und mit kaltem Methanol mehrmals gewaschen. Man erhielt 22,4 g reines β-Apo-8'-carotinal ($E_1^1$ = 2540 λ max. 459 nm, in Cyclohexan). Die Ausbeute betrug somit 60,8 % der Theorie.

c) Wurde anstelle von 1,1-Dimethoxy-2-methyl-4,4-diacetoxi-2-buten das 1,1-[2',2'-Dimethylpropylen-1',3'-dioxi]-2-methyl-4,4-diacetoxi-2-buten eingesetzt, so erhielt man in 52 %iger Ausbeute rohes β-Apo-12'-carotinal und daraus in 57 %iger Ausbeute kristallines reines β-Apo-8'-carotinal.

Die Versuche wurden nicht optimiert. Die erhaltenen kristallinen β-Apo-8'-carotinale entsprachen in allen physikalischen Daten (UV-Spektrum, NMR und C 13 NMR) den Literaturwerten.

**Patentansprüche**

1. 1,1-Dialkoxy-2-methyl-4,4-diacyloxy-2-butene der allgemeinen Formel I

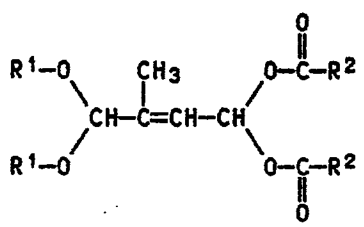

(I)

in der

$R^1$ einen Alkylrest mit 1 bis 5 C-Atomen bedeutet oder aber beide $R^1$ zusammen einen Ethylen- oder Propylenrest, der noch durch $C_1$-$C_4$-Alkylreste substituiert sein kann, bedeuten und

$R^2$ für einen Alkylrest mit 1 bis 5 C-Atomen oder einen Cycloalkylrest mit 5 bis 7 C-Atomen, oder einen aromatischen Rest, vorzugsweise eine Phenylgruppe steht.

2. 1,1-Dialkoxy-2-methyl-4,4-diacyloxy-2-butene der allgemeinen Formel I gemäß Anspruch 1

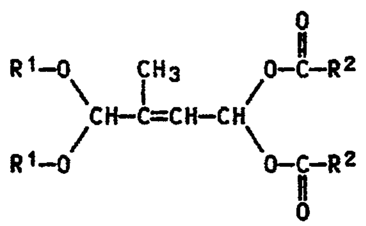

(I),

in der

$R^1$ einen Alkylrest mit 1 bis 3 C-Atomen bedeutet oder beide $R^1$ zusammen einen Ethylenrest oder einen 2,2-Dimethyl-propylenrest bedeuten und

$R^2$ für einen Alkylrest mit 1 bis 3 C-Atomen steht.

3. 1,1-Dimethoxy-2-methyl-4,4-diacetoxy-2-buten.

## Claims

1. A 1,1-dialkoxy-2-methyl-4,4-diacyloxy-2-butene of the formula I

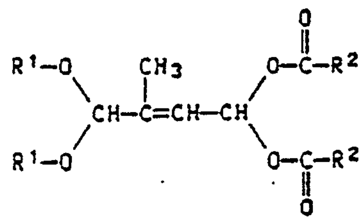

(I)

where $R^1$ is alkyl of 1 to 5 carbon atoms or both radicals $R^1$ together form an ethylene or propylene radical which may furthermore be substituted by $C_1$–$C_4$-alkyl, and $R^2$ is alkyl of 1 to 5 carbon atoms or cycloalkyl of 5 to 7 carbon atoms or an aromatic radical, preferably phenyl.

2. A 1,1-dialkoxy-2-methyl-4,4-diacyloxy-2-butene of the tormula I as claimed in claim 1

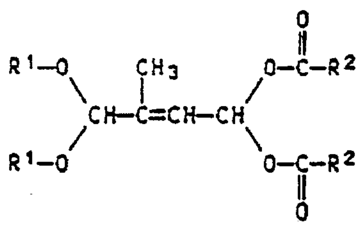

(I)

where $R^1$ is alkyl of 1 to 3 carbon atoms or both radicals $R^1$ together form an ethylene radical or a 2,2-dimethyl-propylene radical and $R^2$ is alkyl of 1 to 3 carbon atoms.

3. 1,1-Dimethoxy-2-methyl-4,4-diacetoxy-2-butene.

## Revendications

1. 1,1-dialcoxy-2-méthyl-4,4-diacyloxy-2-butènes de formule générale I

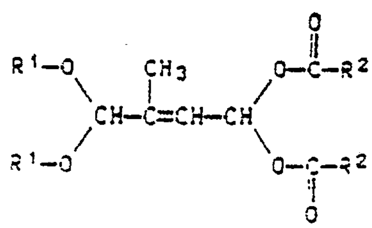

(I),

dans laquelle $R^1$ représente un reste alkyle ayant 1 à 5 atomes C ou bien les deux $R^1$, ensemble, représentent un reste éthylène ou propylène, qui peut encore être substitué par des restes alkyle en $C_1$–$C_4$, et $R^2$est mis pour un reste alkyle ayant 1 à 5 atomes C ou un reste cycloalkyle ayant 5 à 7 atomes C ou un reste aromatique, de préférence un groupe phényle.

2. 1,1-dialcoxy-2-méthyl-4,4-diacyloxy-2-butènes de formule générale I

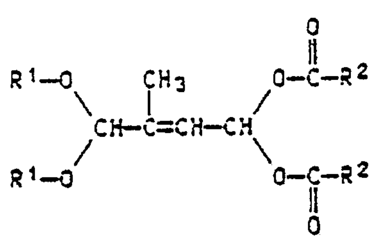

(I),

dans laquelle $R^1$ représente un reste alkyle ayant 1 à 3 atomes C ou bien les deux $R^1$, ensemble, représentent un reste éthylène ou un reste 2,2-diméthyl-propylène et $R^2$ est mis pour un reste alkyle ayant 1 à 3 atomes C.

3. 1,1-diméthoxy-2-méthyl-4,4-diacétoxy-2-butène.